(19) Europäisches Patentamt
European Patent Office
Office européen des brevets

(11) **EP 1 546 757 B1**

(12) **FASCICULE DE BREVET EUROPEEN**

(45) Date de publication et mention de la délivrance du brevet:
**26.11.2008 Bulletin 2008/48**

(21) Numéro de dépôt: **03773770.7**

(22) Date de dépôt: **12.08.2003**

(51) Int Cl.:
***G01S 7/52*** *(2006.01)*

(86) Numéro de dépôt international:
**PCT/FR2003/002516**

(87) Numéro de publication internationale:
**WO 2004/021038 (11.03.2004 Gazette 2004/11)**

(54) **PROCEDE ET DISPOSITIF D'IMAGERIE UTILISANT DES ONDES DE CISAILLEMENT**

ABBILDUNGSVERFAHREN UND EINRICHTUNG MIT VERWENDUNG VON SHEARWELLEN

IMAGING METHOD AND DEVICE EMPLOYING SHEAR WAVES

(84) Etats contractants désignés:
**AT BE BG CH CY CZ DE DK EE ES FI FR GB GR HU IE IT LI LU MC NL PT RO SE SI SK TR**

(30) Priorité: **02.09.2002 FR 0210838**

(43) Date de publication de la demande:
**29.06.2005 Bulletin 2005/26**

(73) Titulaires:
• **CENTRE NATIONAL DE LA RECHERCHE SCIENTIFIQUE (CNRS)**
**75794 Paris Cedex 16 (FR)**
• **Universite Paris 7-Denis Diderot**
**75251 Paris Cedex 05 (FR)**

(72) Inventeurs:
• **FINK, Mathias**
**F-92190 Meudon (FR)**
• **TANTER, Mickael**
**F-75006 Paris (FR)**

(74) Mandataire: **Burbaud, Eric**
**Cabinet Plasseraud**
**52 rue de la Victoire**
**75440 Paris Cedex 09 (FR)**

(56) Documents cités:
FR-A- 2 791 136          FR-A- 2 815 717
US-A- 5 010 885          US-A- 5 276 654

## Description

La présente invention est relative aux procédés et dispositifs d'imagerie utilisant des ondes de cisaillement.

Plus particulièrement, l'invention concerne un procédé d'imagerie utilisant des ondes de cisaillement pour observer un milieu viscoélastique diffusant qui contient des particules réfléchissant les ondes ultrasonores de compression, procédé comprenant :

(a) une étape d'excitation au cours de laquelle on génère une onde élastique de cisaillement dans le milieu viscoélastique,

(b) une étape d'observation au cours de laquelle on observe la propagation de l'onde de cisaillement simultanément en une multitude de points d'un champ d'observation dans le milieu viscoélastique, cette étape d'observation comprenant les sous-étapes suivantes :

(b1) faire émettre dans le milieu viscoélastique, par un réseau de transducteurs commandés indépendamment les uns des autres, une succession de tirs d'ondes ultrasonores de compression non focalisées à une cadence d'au moins 500 tirs par seconde,

(b2) faire détecter et enregistrer en temps réel des signaux acoustiques reçus du milieu viscoélastique 2, comprenant les échos générés par les ondes ultrasonores de compression non focalisées en interagissant avec les particules réfléchissantes dudit milieu viscoélastique,

(c) et au moins une étape de traitement au cours de laquelle :

(c1) on traite les signaux acoustiques successifs reçus du milieu viscoélastique au cours de la sous-étape (b2) pour déterminer des images de propagation successives de l'onde de cisaillement,

(c2) et pour déterminer au moins un paramètre de mouvement du milieu viscoélastique en différents points du champ d'observation.

On obtient ainsi un "film" illustrant clairement la propagation de l'onde de cisaillement dans le milieu viscoélastique, qui peut permettre une analyse qualitative et/ou quantitative pour repérer des zones de dureté différente du reste du milieu viscoélastique ou des zones ayant un temps de relaxation différent du reste du milieu viscoélastique.

Le document WO-A-00/55 616 décrit un exemple d'un tel procédé, dans lequel on génère l'onde de cisaillement à la surface du milieu viscoélastique. Ce procédé donne toute satisfaction notamment pour imager des zones situées relativement près de la surface du milieu viscoélastique. Mais ce procédé connu ne permet pas d'observer certaines zones du milieu viscoélastique, notamment :

- des zones suffisamment profondes pour ne pas pouvoir être atteintes par des ondes de cisaillement générées en surface (les ondes de cisaillement s'atténuent rapidement),
- et des zones d'ombre masquées par des obstacles (notamment des parties du squelette d'un patient ou des zones liquides telles que des kystes liquides) qui gênent la propagation des ondes de cisaillement.

De plus, si le champ d'observation est partiellement dans une zone d'ombre, il peut être nécessaire de déplacer le dispositif générateur des ondes de cisaillement en cours d'observation, ce qui est fastidieux pour l'utilisateur.

Enfin, le dispositif générateur des ondes de cisaillement est relativement lourd et complique le dispositif.

La présente invention a notamment pour but de pallier ces inconvénients.

A cet effet, selon l'invention, un procédé du genre en question est **caractérisé en ce qu'**au cours de l'étape d'excitation (a), on fait générer l'onde élastique de cisaillement en faisant émettre au moins une onde ultrasonore focalisée dans le milieu viscoélastique par ledit réseau de transducteurs, la focalisation et la chronologie de ladite onde ultrasonore focalisée, ainsi que la chronologie desdites ondes ultrasonores non focalisées, étant adaptées pour qu'au moins certaines desdites ondes ultrasonores non focalisées parviennent dans le champ d'observation lors de la propagation de l'onde de cisaillement dans ce champ d'observation.

Ainsi, le même réseau de transducteurs permet à la fois d'engendrer l'onde élastique de cisaillement de façon choisie dans le champ d'observation, et d'observer ensuite cette propagation, grâce au fait que le dispositif d'imagerie est adapté pour générer soit des ondes ultrasonores focalisées permettant de générer l'onde élastique de cisaillement, soit des ondes ultrasonores non focalisées permettant d'observer la propagation de l'onde de cisaillement, et grâce au choix judicieux :

- de la chronologie de ces différentes émissions,
- et du ou des points de focalisation de l'onde ultrasonore focalisée.

Le procédé d'imagerie selon l'invention est donc aisé à mettre en oeuvre pour un utilisateur, avec un dispositif relativement simple et léger. L'invention est donc d'un très faible coût de revient en comparaison de techniques concurrentes telles que l'IRM, et elle permet le cas échéant de constituer des systèmes d'imagerie ambulatoires utilisables tant en imagerie préopératoire qu'en imagerie postopératoire, voire en imagerie per-opératoire.

A titre d'exemple, dans des applications médicales, le procédé selon l'invention peut permettre de repérer efficacement des zones cancéreuses dans les tissus d'un patient. La propagation des ondes de cisaillement s'y déroule en effet très différemment des zones voisines. Ce repérage s'effectue beaucoup plus facilement que par une observation classique par simple échographie ultrasonore, puisque la propagation des ondes de cisaillement est fonction du module de cisaillement du milieu, lui-même très variable entre une zone de tissus sains et une zone de tissus cancéreux : le module de cisaillement varie typiquement dans un rapport de 1 à 30 entre une zone saine et une zone cancéreuse, alors que le module de compression, qui régit la propagation des ondes acoustiques de compression utilisées dans l'échographie ultrasonore, varie seulement de l'ordre de 5% entre un tissu sain et un tissu cancéreux.

De même, on peut ainsi repérer des zones nécrosées au sein d'un tissu, par exemple des zones tumorales ayant subi un traitement par hyperthermie aux ultrasons, notamment pour évaluer l'efficacité de ce traitement par hyperthermie.

Une autre application possible de l'invention concerne l'évaluation quantitative du degré de fibrose du foie, qui est un paramètre important dans les pathologies du foie, notamment l'hépatite C.

On notera que l'invention permet de générer l'onde de cisaillement, et d'observer sa propagation, y compris à travers une zone liquide ou une barrière osseuse masquant totalement ou partiellement le champ d'observation (crâne, cage thoracique, etc.), puisqu'il est possible de focaliser des ondes ultrasonores au travers de telles barrières (voir notamment le document WO-A-02/32316 ou la demande de brevet français n° 02 10682 du 28 août 2002).

Dans des modes de réalisation préférés du procédé selon l'invention, on peut éventuellement avoir recours en outre à l'une et/ou à l'autre des dispositions suivantes :

- au cours de la sous-étape (b2), pour déterminer ledit paramètre de mouvement, on compare plusieurs images successives de propagation (par exemple par corrélation, Doppler, etc.) avec une même image de référence du milieu viscoélastique, cette image de référence était déterminée en tirant au moins une onde ultrasonore de compression non focalisée dans ledit milieu viscoélastique puis en détectant et en enregistrant des échos générés par ladite onde ultrasonore de compression non focalisée lorsqu'elle interagit avec les particules réfléchissantes du milieu viscoélastique (on améliore ainsi la précision de la mesure du paramètre de mouvement (par exemple le déplacement) du milieu viscoélastique, notamment pour de faibles amplitudes de mouvement (typiquement moins de 30 $\mu$m avec la technique d'excitation par ondes acoustiques utilisée ici) ;

- l'étape (a) est précédée d'une étape d'observation initiale (a0) au cours de laquelle on tire au moins une onde ultrasonore de compression non focalisée puis on détecte et on enregistre des échos générés par ladite onde ultrasonore de compression non focalisée en interagissant avec les particules réfléchissantes du milieu viscoélastique, ces échos correspondant (directement ou indirectement) à une image initiale du milieu viscoélastique, et au cours de la sous-étape (b2) ladite image initiale constitue ladite image de référence pour traiter au moins certaines des images successives de déplacement ;

- au cours de l'étape d'observation initiale (a0), on tire successivement plusieurs ondes ultrasonores de compression non focalisées puis on détecte et on enregistre des échos générés par chaque onde ultrasonore de compression non focalisée en interagissant avec les particules réfléchissantes du milieu viscoélastique, ces échos correspondant (directement ou indirectement) à plusieurs images préliminaires successives du milieu viscoélastique, et on détermine ladite image initiale du milieu viscoélastique en combinant lesdites images préliminaires successives ;

- ledit paramètre de mouvement est un déplacement du milieu viscoélastique ;

- l'onde ultrasonore focalisée émise au cours de l'étape d'excitation (a) présente une fréquence f comprise entre 0,5 et 15 MHz et est émise pendant une durée k/f en secondes, où k est un entier compris entre 50 et 5000 et f est exprimé en Hz ;

- l'onde ultrasonore focalisée émise au cours de l'étape d'excitation (a) présente une fréquence comprise entre 0,5 et 15 MHz et est émise pendant une succession de périodes d'émission séparées par des périodes de repos, les périodes d'émission se succédant à une cadence comprise entre 10 et 1000 émissions par seconde ;

- l'onde ultrasonore focalisée émise au cours de l'étape d'excitation (a) est une combinaison linéaire (notamment une somme) de deux signaux monochromatiques de fréquences respectives f1 et f2 telles que 20 Hz $\leq$ |f1 - f2| $\leq$ 1000 Hz ;

- l'onde ultrasonore focalisée émise au cours de l'étape d'excitation (a) est focalisée simultanément en plusieurs points ;

- l'étape (c) de traitement d'images est suivie (immédiatement ou non) par une étape (d) de cartographie au cours de laquelle, à partir d'une évolution du paramètre de mouvement au cours du temps, on calcule au moins un paramètre de propagation de l'onde de cisaillement en au moins certains points du champ d'observation pour déterminer ainsi une cartographie dudit paramètre de propagation dans le champ d'observation ;

- le paramètre de propagation de l'onde de cisaillement qui est calculé au cours de l'étape de cartographie est choisi parmi la vitesse des ondes de cisaillement, le module de cisaillement, le module d'Young, l'atténuation des ondes de cisaillement, l'élasticité de cisaillement, la viscosité de cisaillement et le temps de relaxation mécanique ;
- on répète successivement les étapes (a) à (d) en émettant des ondes ultrasonores différentes au cours des étapes d'excitation (a) successives, puis on combine les différentes cartographies obtenues au cours des étapes de cartographie (d) successives, pour calculer une cartographie synthétique du champ d'observation.

Par ailleurs, l'invention a également pour objet un dispositif d'imagerie utilisant des ondes de cisaillement pour observer un milieu viscoélastique diffusant qui contient des particules réfléchissant les ondes ultrasonores de compression, ce dispositif comprenant un réseau de transducteurs commandés indépendamment les uns des autres par au moins une unité centrale électronique adaptée pour :

- faire générer au moins une onde élastique de cisaillement dans le milieu viscoélastique,
- observer la propagation de l'onde de cisaillement simultanément en une multitude de points d'un champ d'observation dans le milieu viscoélastique, en faisant émettre dans le milieu viscoélastique, par ledit réseau de transducteurs, une succession de tirs d'ondes ultrasonores de compression non focalisées, à une cadence d'au moins 500 tirs par seconde, puis en faisant détecter en temps réel par ledit réseau de transducteurs, et pour enregistrer en temps réel, des signaux acoustiques reçus du milieu viscoélastique, comprenant les échos générés par les ondes ultrasonores de compression non focalisées en interagissant avec les particules réfléchissantes dudit milieu viscoélastique,
- et traiter les signaux acoustiques successifs reçus du milieu viscoélastique pour déterminer des images de propagation successives de l'onde de cisaillement, puis déterminer au moins un paramètre de mouvement du milieu viscoélastique en différents points du champ d'observation, caractérisé en ce que l'unité centrale électronique est adaptée pour faire générer l'onde élastique de cisaillement en faisant émettre au moins une onde ultrasonore focalisée dans le milieu viscoélastique par ledit réseau de transducteurs, la focalisation et la chronologie de ladite onde ultrasonore focalisée, ainsi que la chronologie desdites ondes ultrasonores non focalisées, étant adaptées pour que lesdites ondes ultrasonores non focalisées parviennent dans le champ d'observation lors de la propagation de l'onde de cisaillement dans ce champ d'observation.

D'autres caractéristiques et avantages de l'invention apparaîtront au cours de la description suivante d'une de ses formes de réalisation, donnée à titre d'exemple non limitatif, en regard du dessin joint.

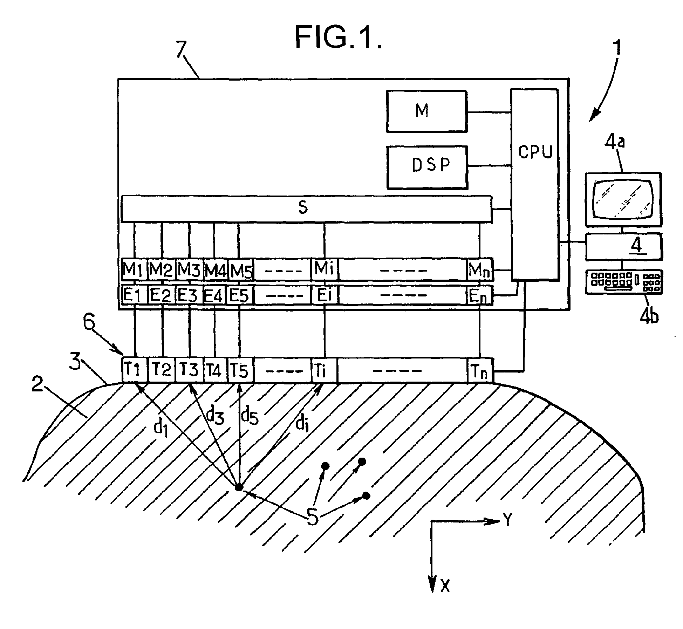

Sur le dessin, la figure 1 est une vue schématique d'un dispositif d'imagerie par ondes de cisaillement selon une forme de réalisation de l'invention.

Le dispositif d'imagerie 1 représenté sur la figure 1 est destiné à étudier la propagation des ondes élastiques de cisaillement dans un milieu viscoélastique 2 qui est diffusant vis à vis des ondes ultrasonores de compression, et qui peut être par exemple :

- un corps inerte, notamment dans le cas du contrôle de qualité pour des applications industrielles,
- ou un corps vivant, par exemple une partie du corps d'un patient, dans le cas des applications médicales.

Ces mouvements sont suivis par exemple au moyen d'un micro-ordinateur 4 (comprenant au moins une interface d'entrée 4b telle qu'un clavier ou autre, et une interface de sortie 4a telle qu'un écran ou autre) ou toute autre unité centrale électronique, qui fait envoyer dans le milieu 2, à partir de sa surface extérieure 3, des ondes ultrasonores de compression qui interagissent avec les particules diffusantes 5 contenues dans le milieu 2, lesquelles particules sont réfléchissantes pour les ondes ultrasonores de compression. Les particules 5 peuvent être constituées par toute hétérogénéité du milieu 2, et notamment, lorsqu'il s'agit d'une application médicale, par des particules de collagène présentes dans les tissus humains (ces particules forment sur les images échographiques des points connus sous le terme "speckle").

Pour observer la propagation de l'onde de cisaillement, on utilise une sonde ultrasonore 6 disposée contre la surface extérieure 3 du milieu observé 2. Cette sonde envoie, selon un axe X, des impulsions d'ondes ultrasonores de compression du type de celles couramment utilisées en échographie, à une fréquence comprise par exemple entre 0,5 et 100 MHz et de préférence entre 0,5 et 15 MHz, par exemple de l'ordre de 4 MHz.

La sonde ultrasonore 6 est constituée par un réseau de n transducteurs ultrasonores T1, T2, ..., Ti, ..., Tn, n étant un nombre entier n au moins égal à 1.

Cette sonde 6 peut se présenter par exemple sous la forme d'une barrette linéaire pouvant comprendre par exemple n = 128 transducteurs alignés selon un axe Y perpendiculaire à l'axe X. Mais la sonde en question pourrait être également un réseau bidimensionnel (plan ou non) de transducteurs.

Les transducteurs T1, T2, ... Tn sont commandés indépendamment les uns des autres par le micro-ordinateur 4, éventuellement par l'intermédiaire d'une unité centrale CPU qui est contenue par exemple dans une baie électronique 7 reliée par un câble souple à la sonde 6. Les transducteurs T1-Tn peuvent ainsi émettre sélectivement :

- soit une onde ultrasonore de compression "plane" (c'est à dire en l'occurrence une onde dont le front d'onde est rectiligne dans le plan X, Y) ou tout autre type d'onde non focalisée éclairant l'ensemble du champ d'observation dans le milieu 2, par exemple une onde générée en faisant émettre des signaux acoustiques aléatoires par les différents transducteurs T1-Tn,
- soit une onde ultrasonore de compression focalisée en un ou plusieurs points du milieu 2.

Pour observer la propagation de l'onde de cisaillement dans le milieu 2, on procède en plusieurs étapes successives :

(a) une étape d'excitation au cours de laquelle le micro-ordinateur 4 fait générer une onde élastique de cisaillement dans le milieu viscoélastique 2, en faisant émettre au moins une onde ultrasonore focalisée dans le milieu viscoélastique par la sonde 6,
(b) une étape d'observation au cours de laquelle on observe la propagation de l'onde de cisaillement simultanément en une multitude de points du champ d'observation dans le milieu viscoélastique 2, cette étape d'observation comprenant les sous-étapes suivantes :

(b1) le micro-ordinateur 4 fait émettre dans le milieu viscoélastique, par la sonde 6 une succession de tirs d'ondes ultrasonores de compression non focalisées à une cadence d'au moins 500 tirs par seconde (la focalisation et la chronologie de l'onde ultrasonore focalisée émise à l'étape (a), ainsi que la chronologie desdites ondes ultrasonores non focalisées, sont adaptées pour qu'au moins certaines desdites ondes ultrasonores non focalisées parviennent dans le champ d'observation lors de la propagation de l'onde de cisaillement dans ce champ d'observation, pour au moins certaines émissions d'onde ultrasonore non focalisée),
(b2) le micro-ordinateur 4 fait détecter par la sonde 6 et enregistrer en temps réel des signaux acoustiques reçus du milieu viscoélastique 2, comprenant les échos générés par les ondes ultrasonores de compression non focalisées en interagissant avec les particules réfléchissantes 5 du milieu viscoélastique, ces échos correspondant (directement ou indirectement) à des images successives de déplacement du milieu viscoélastique,

(c) et au moins une étape de traitement au cours de laquelle :

(c1) le micro-ordinateur 4 traite les signaux acoustiques successifs reçus du milieu viscoélastique 2 au cours de la sous-étape (b2) pour déterminer des images de propagation successives,
(c2) et le micro-ordinateur 4 détermine au moins un paramètre de mouvement du milieu viscoélastique 2 en différents points du champ d'observation.

L'onde ultrasonore focalisée émise au cours de l'étape d'excitation (a) peut être une onde monochromatique de fréquence f comprise entre 0,5 et 15 MHz, par exemple environ 4 MHz, émise pendant une durée k/f en secondes, où k est un entier compris entre 50 et 5000 (par exemple de l'ordre de 500) et f est exprimé en Hz. Eventuellement, une telle onde peut être émise pendant une succession de périodes d'émission séparées par des périodes de repos, les périodes d'émission se succédant à une cadence comprise entre 10 et 1000 émissions par seconde.

En variante, l'onde ultrasonore focalisée émise au cours de l'étape d'excitation (a) est une combinaison linéaire (notamment une somme) de deux signaux monochromatiques de fréquences respectives f1 et f2 telles que $20\ Hz \leq |f1 - f2| \leq 1000\ Hz$, ce qui produit une onde modulée en amplitude avec une fréquence de modulation |f1 - f2|.

Par ailleurs, l'onde ultrasonore focalisée émise au cours de l'étape d'excitation (a) peut éventuellement être focalisée, simultanément ou non, en plusieurs points, de façon que l'onde de cisaillement générée présente la forme souhaitée (par exemple, on peut ainsi générer une onde de cisaillement plane, ou au contraire une onde de cisaillement focalisée) et illumine les zones souhaitées du milieu 2.

Au cours de l'étape (b1), qui peut durer par exemple moins d'une seconde, on peut émettre les ondes ultrasonores de compression non focalisées, à une cadence comprise entre 500 et 10 000 tirs par seconde et de préférence comprise entre 1000 et 5 000 tirs par seconde (cette cadence est limitée par le temps d'aller-retour de l'onde de compression dans le milieu 2, donc par l'épaisseur du milieu 2 dans la direction X : il faut en effet que tous les échos générés par l'onde de compression aient été reçus par la sonde 6 avant d'envoyer une nouvelle onde de compression).

Chaque onde ultrasonore de compression non focalisée se propage dans le milieu 2 avec une vitesse de propagation beaucoup plus élevée que les ondes de cisaillement (par exemple de l'ordre de 1500 m/s dans le corps humain), et interagit avec les particules réfléchissantes 5, ce qui génère des échos ou autres perturbations analogues du signal, connus en soi sous le nom de "bruits de speckle" dans le domaine de l'échographie.

Ces "bruits de speckle" sont captés par les transducteurs T1, ... , Tn au cours de la sous-étape (b2), après chaque tir d'onde ultrasonore de compression non focalisée. Le signal sij(t) ainsi capté par chaque transducteur Ti après le tir n° j est tout d'abord échantillonné à haute fréquence (par exemple de 30 à 100 MHz) et numérisé en temps réel (par exemple sur 12 bits) par un échantillonneur appartenant à la baie 7 et relié à ce transducteur, respectivement E1, E2, ... En.

Le signal sij(t) ainsi échantillonné et numérisé est ensuite mémorisé, également en temps réel, dans une mémoire Mi appartenant à la baie 7 et propre au transducteur Ti.

Chaque mémoire Mi présente par exemple une capacité de l'ordre de 128 Mo, et contient l'ensemble des signaux sij(t) reçus successivement pour les tirs j = 1 à p.

En temps différé, après la mémorisation de tous les signaux sij (t) correspondant à une même propagation d'onde de cisaillement, l'unité centrale CPU fait retraiter ces signaux par un circuit sommateur S appartenant à la baie 7 (ou bien elle effectue elle-même ce traitement, ou encore ledit traitement peut être effectué dans le micro-ordinateur 4), par un processus classique de formation de voies correspondant à la sous-étape (c1).

On génère ainsi des signaux Sj (x, y) qui correspondent chacun à l'image du champ d'observation après le tir n° j (dans le cas où l'onde ultrasonore non focalisée est une onde plane).

Par exemple, on peut déterminer un signal Sj (t) par la formule suivante :

$$Sj(t) = \sum_{i=1}^{n} \alpha_i(x, y).sij[t(x, y) + d_i(x, y) / V]$$

où :

- sij est le signal brut perçu par le transducteur n° i après le tir d'onde ultrasonore de compression n° j,
- t(x,y) est le temps mis par l'onde ultrasonore de compression pour atteindre le point du champ d'observation de coordonnées (x,y), avec t = 0 au début du tir n° j,
- di(x,y) est la distance entre le point du champ d'observation de coordonnées (x,y) et le transducteur n° i, ou une approximation de cette distance,
- V est la vitesse moyenne de propagation des ondes acoustiques ultrasonores de compression dans le milieu viscoélastique observé.
- et $\alpha i(x,y)$ est un coefficient de pondération tenant compte de lois d'apodisation (en pratique, on pourra dans de nombreux cas considérer que $\alpha i(x,y)=1$).

La formule ci-dessus s'applique mutatis mutandis lorsque le champ d'observation est à 3 dimensions (réseau bidimensionnel de transducteurs), en remplaçant les coordonnées spatiales (x,y) par (x,y,z).

Après l'étape éventuelle de formation de voies, l'unité centrale CPU mémorise dans une mémoire centrale M appartenant à la baie 7 les signaux d'images Sj(x,y) ou Sj(x) ou Sj(x,y,z), qui correspondent chacun au tir n° j. Ces signaux peuvent également être mémorisés dans le micro-ordinateur 4 lorsqu'il effectue lui-même le traitement d'image.

Ces images sont ensuite traitées en temps différé à la sous-étape (c2), par corrélation et avantageusement par intercorrélation soit deux à deux, soit de préférence avec une image de référence qui peut être :

- soit une image de déplacement précédemment déterminée comme expliqué ci-dessus et utilisée comme image de référence pour les images de déplacement ultérieures (ou pour un nombre limité d'images de déplacement ultérieures, par exemple 30 images de déplacement),
- soit déterminée au cours d'une étape préliminaire d'observation initiale (a0), comme les images successives de déplacement susmentionnées, en faisant émettre une ou plusieurs ondes ultrasonores non focalisées par la sonde 6 avant l'étape d'excitation (a) qui génère l'onde de cisaillement (lorsque plusieurs ondes ultrasonores de compression non focalisées sont ainsi émises avant la phase d'excitation, on enregistre des échos générés par chaque onde ultrasonore de compression non focalisée en interagissant avec les particules réfléchissantes du milieu viscoélastique, ces échos correspondant à plusieurs images préliminaires successives du milieu viscoélastique, et on détermine ladite image initiale du milieu viscoélastique en combinant lesdites images préliminaires successives et notamment en moyennant les valeurs des pixels desdites images préliminaires).

L'intercorrélation susmentionnée peut être réalisée par exemple dans un circuit électronique spécialisé DSP appartenant à la baie 7, ou être programmée dans l'unité centrale CPU ou dans le micro-ordinateur 4.

Au cours de ce processus d'intercorrélation, on maximise une fonction d'intercorrélation <Sj(x,y),Sj+1(x,y)> afin de déterminer le déplacement subi par chaque particule 5 donnant lieu à un écho ultrasonore.

Des exemples de tels calculs d'intercorrélation sont donnés dans l'état de la technique, notamment par O'Donnell et al. ("Internal displacement and strain imaging using speckle tracking", IEEE transactions on ultrasonic, ferroelectrics, and frequency control, vol. 41, n° 3, mai 1994, p. 314-325) et par Ophir et al. ("Elastography : a quantitative method for imaging the elasticity of biological tissues", Ultrasonic imag., vol. 13, p.111-134, 1991).

On obtient ainsi un ensemble de vecteurs déplacements $\vec{u}(\vec{r},t)$ engendrés par les ondes de cisaillement en chaque position $\vec{r}$ du milieu 2 sous l'effet de l'onde de cisaillement (ces vecteurs déplacements peuvent éventuellement être réduits à une seule composante dans l'exemple considéré ici).

Cet ensemble de vecteurs déplacements est stocké dans la mémoire M ou dans le micro-ordinateur 4 et peut par exemple être visualisé, notamment au moyen de l'écran 4a du micro-ordinateur, sous la forme d'un film ralenti où la valeur des déplacements est illustrée par un paramètre optique tel que par un niveau de gris ou par un niveau chromatique.

On visualise ainsi parfaitement les différences de propagation de l'onde de cisaillement entre les zones de caractéristiques différentes du milieu 2, par exemple les tissus sains et les tissus cancéreux dans le cas d'une application médicale.

Ce film de propagation de l'onde de cisaillement est en outre superposable avec une image échographique classique, qui peut être générée également par le dispositif 1 décrit ci-dessus.

Par ailleurs, il est également possible de calculer non pas les déplacements de chaque point du milieu observé 2, mais les déformations du milieu 2, c'est à dire des vecteurs dont les composantes sont les dérivées des composantes des vecteurs déplacements respectivement par rapport aux variables d'espace (coordonnées selon X, Y dans l'exemple considéré). Ces vecteurs de déformation sont utilisables comme les vecteurs déplacements pour visualiser clairement la propagation de l'onde de cisaillement sous la forme d'un film, et présentent en outre l'avantage de s'affranchir des déplacements de la sonde 6 par rapport au milieu observé 2.

A partir des champs de déplacements ou de déformations, le micro-ordinateur 4 peut avantageusement procéder ensuite à une étape de cartographie (d) au cours de laquelle, à partir de l'évolution du paramètre de mouvement (déplacement ou déformation) au cours du temps dans le champ d'observation X, Y (ou X, Y, Z dans le cas d'un réseau bidimensionnel de transducteurs), on calcule au moins un paramètre de propagation de l'onde de cisaillement, soit en certains points du champ d'observation choisis par l'utilisateur à partir du micro-ordinateur 4, soit dans tout le champ d'observation.

Le paramètre de propagation de l'onde de cisaillement qui est calculé au cours de l'étape de cartographie est choisi par exemple parmi : la vitesse Cs des ondes de cisaillement, le module de cisaillement $\mu$ ou le module d'Young E=3$\mu$, l'atténuation $\alpha$ des ondes de cisaillement, l'élasticité dé cisaillement $\mu$1, la viscosité de cisaillement $\mu$2, ou le temps de relaxation mécanique $\tau$s des tissus.

Par exemple, on peut calculer en différents points du champ d'observation :

- la valeur de la célérité Cs de l'onde de cisaillement, qui donne accès à la dureté des tissus,
- la valeur du temps de relaxation mécanique $\tau$s des tissus, caractéristiques de la viscosité locale du milieu.

Pour cela, on utilise l'équation de propagation (1) suivante, à laquelle obéissent les déplacements $\vec{u}(\vec{r},t)$ engendrés par les ondes de cisaillement en chaque position $\vec{r}$ du milieu :

$$\rho \frac{\partial^2 \vec{u}(\vec{r},t)}{\partial t^2} = c_s^2 \, (1 + \tau_s \frac{\partial \cdot}{\partial t}).\vec{\nabla}^2 \vec{u}(\vec{r},t)$$

$$(1)$$

où $\rho$ est la densité des tissus, $\tau$S est le temps de relaxation mécanique des tissus et cS est la célérité de l'onde de cisaillement, directement reliée au module d'Young E des tissus par la relation :

$$c_s = \sqrt{\frac{E}{3\rho}}$$

$$(2)$$

Dans le domaine de Fourier, l'équation d'onde (1) ci-dessus peut s'écrire :

$$\omega^2 \rho\, U(\vec{r},\omega) = c_S{}^2\,(1 + j\omega\tau).\Delta U(\vec{r},\omega) \qquad (3)$$

où $U(\vec{r},\omega)$ est la transformé de Fourier du champ de déplacement $\vec{u}(\vec{r},t)$ mesuré en chaque point et $\Delta U(\vec{r},\omega)$ est la transformée de Fourier du laplacien spatial de ce champ $\vec{u}(\vec{r},t)$. Etant donné que $\omega\tau S \ll 1$, on a donc accès à une expression simplifiée :

$$c_S{}^2 = \omega^2 \rho\, \frac{U(\vec{r},\omega)}{\Delta U(\vec{r},\omega)} \qquad (4)$$

$$\tau_S = \frac{1}{\omega}\tan\!\left(\Psi\!\left(\frac{U(\vec{r},\omega)}{\Delta U(\vec{r},\omega)}\right)\right) \qquad (5)$$

où $\Psi(x)$ est la phase de la variable complexe x.

Les fonctions $U(\vec{r},\omega)$ et $\Delta U(\vec{r},\omega)$ étant connues en chaque point de l'image échographique, il est donc possible de mesurer en tout point de l'espace le module d'Young et le temps de relaxation mécanique des tissus, en établissant ainsi une cartographie de ces deux paramètres.

De plus, les équations (4) et (5) étant vraies à chaque fréquence, le calcul de cS et $\tau$S peut être avantageusement moyenné sur l'ensemble de la bande des fréquences portées par l'onde de cisaillement, améliorant ainsi fortement la qualité de la cartographie réalisée. A cet effet, on peut utiliser les formules suivantes :

$$c_S{}^2 = \frac{1}{\omega_1 - \omega_0}\int_{\omega_0}^{\omega_1} \omega^2 \rho\, \frac{U(\vec{r},\omega)}{\Delta U(\vec{r},\omega)}\, d\omega \qquad (6)$$

$$\tau_S = \frac{1}{\omega_1 - \omega_0}\int_{\omega_0}^{\omega_1} \frac{1}{\omega}\tan\!\left(\Psi\!\left(\frac{U(\vec{r},\omega)}{\Delta U(\vec{r},\omega)}\right)\right) d\omega \qquad (7)$$

où $\omega 0$ et $\omega 1$ sont les fréquences minimales et maximales que porte l'onde de cisaillement

Le mode de calcul serait le même en utilisant non plus les déplacements, mais les déformations du milieu observé 2.

Par ailleurs, on peut avantageusement établir successivement plusieurs cartographies des paramètres de propagation souhaités, par exemple cS et $\tau$S, en générant successivement des ondes de cisaillement différentes, obtenues par exemple en émettant des ondes ultrasonores de compression focalisées successivement en plusieurs points ou ayant des formes d'ondes différentes. On peut ensuite combiner les différentes cartographies obtenues, par exemple en les moyennant, de façon à obtenir une cartographie synthétique plus riche et plus précise.

**Revendications**

**1.** Procédé d'imagerie utilisant des ondes de cisaillement pour observer un milieu viscoélastique diffusant (2) qui contient des particules (5) réfléchissant les ondes ultrasonores de compression, procédé comprenant :

(a) une étape d'excitation au cours de laquelle on génère une onde élastique de cisaillement dans le milieu viscoélastique (2),

(b) une étape d'observation au cours de laquelle on observe la propagation de l'onde de cisaillement simultanément en une multitude de points d'un champ d'observation dans le milieu viscoélastique (2), cette étape d'observation comprenant les sous-étapes suivantes :

(b1) faire émettre dans le milieu viscoélastique (2), par un réseau de transducteurs (6) commandés indépendamment les uns des autres, une succession de tirs d'ondes ultrasonores de compression non focalisées à une cadence d'au moins 500 tirs par seconde,

(b2) faire détecter et enregistrer en temps réel des signaux acoustiques reçus du milieu viscoélastique (2), comprenant les échos générés par les ondes ultrasonores de compression non focalisées en interagissant avec les particules réfléchissantes (5) dudit milieu viscoélastique,

(c) et au moins une étape de traitement au cours de laquelle :

(c1) on traite les signaux acoustiques successifs reçus du milieu viscoélastique (2) au cours de la sous-étape (b2) pour déterminer des images de propagation successives de l'onde de cisaillement,

(c2) et on détermine au moins un paramètre de mouvement du milieu viscoélastique (2) en différents points du champ d'observation,

**caractérisé en ce qu'**au cours de l'étape d'excitation (a), on fait générer l'onde élastique de cisaillement en faisant émettre au moins une onde ultrasonore focalisée dans le milieu viscoélastique (2) par ledit réseau de transducteurs (6), la focalisation et la chronologie de ladite onde ultrasonore focalisée, ainsi que la chronologie desdites ondes ultrasonores non focalisées, étant adaptées pour qu'au moins certaines desdites ondes ultrasonores non focalisées parviennent dans le champ d'observation lors de la propagation de l'onde de cisaillement dans ce champ d'observation, pour au moins certaines émissions d'onde ultrasonore non focalisée.

2. Procédé selon la revendication 1, dans lequel, au cours de la sous-étape (b2), pour déterminer ledit paramètre de mouvement, on compare plusieurs images successives de propagation avec une même image de référence du milieu viscoélastique (2), cette image de référence était déterminée en tirant au moins une onde ultrasonore de compression non focalisée dans ledit milieu viscoélastique puis en détectant et en enregistrant des échos générés par ladite onde ultrasonore de compression non focalisée lorsqu'elle interagit avec les particules réfléchissantes (5) du milieu viscoélastique.

3. Procédé selon la revendication 2, dans lequel l'étape (a) est précédée d'une étape d'observation initiale (a0) au cours de laquelle on tire au moins une onde ultrasonore de compression non focalisée puis on détecte et on enregistre des échos générés par ladite onde ultrasonore de compression non focalisée en interagissant avec les particules réfléchissantes (5) du milieu viscoélastique, ces échos correspondant à une image initiale du milieu viscoélastique, et au cours de la sous-étape (b2) ladite image initiale constitue ladite image de référence pour traiter au moins certaines des images successives de déplacement.

4. Procédé selon la revendication 3, dans lequel, au cours de l'étape d'observation initiale (a0), on tire successivement plusieurs ondes ultrasonores de compression non focalisées puis on détecte et on enregistre des échos générés par chaque onde ultrasonore de compression non focalisée en interagissant avec les particules réfléchissantes (5) du milieu viscoélastique, ces échos correspondant à plusieurs images successives du milieu viscoélastique, et on détermine ladite image initiale du milieu viscoélastique en combinant lesdites images successives.

5. Procédé selon l'une quelconque des revendications précédentes, dans lequel ledit paramètre de mouvement est un déplacement du milieu viscoélastique (2).

6. Procédé selon l'une quelconque des revendications précédentes, dans lequel l'onde ultrasonore focalisée émise au cours de l'étape d'excitation (a) présente une fréquence f comprise entre 0,5 et 15 MHz et est émise pendant une durée k/f en secondes, où k est un entier compris entre 50 et 5000 et f est exprimé en Hz.

7. Procédé selon l'une quelconque des revendications 1 à 5, dans lequel l'onde ultrasonore focalisée émise au cours de l'étape d'excitation (a) présente une fréquence comprise entre 0,5 et 15 MHz et est émise pendant une succession de périodes d'émission séparées par des périodes de repos, les périodes d'émission se succédant à une cadence comprise entre 10 et 1000 émissions par seconde.

**8.** Procédé selon l'une quelconque des revendications 1 à 5, dans lequel l'onde ultrasonore focalisée émise au cours de l'étape d'excitation (a) est une combinaison linéaire (notamment une somme) de deux signaux monochromatiques de fréquences respectives f1 et f2 telles que 20 Hz ≤ |f1 - f2| ≤ 1000 Hz.

**9.** Procédé selon l'une quelconque des revendications précédentes, dans lequel l'onde ultrasonore focalisée émise au cours de l'étape d'excitation (a) est focalisée simultanément en plusieurs points.

**10.** Procédé selon l'une quelconque des revendications précédentes, dans lequel l'étape (c) de traitement d'images est suivie par une étape (d) de cartographie au cours de laquelle, à partir d'une évolution du paramètre de mouvement au cours du temps, on calcule au moins un paramètre de propagation de l'onde de cisaillement en au moins certains points du champ d'observation pour déterminer ainsi une cartographie dudit paramètre de propagation dans le champ d'observation.

**11.** Procédé selon la revendication 10, dans lequel le paramètre de propagation de l'onde de cisaillement qui est calculé au cours de l'étape de cartographie (d) est choisi parmi la vitesse des ondes de cisaillement, le module de cisaillement, le module d'Young, l'atténuation des ondes de cisaillement, l'élasticité de cisaillement, la viscosité de cisaillement et le temps de relaxation mécanique.

**12.** Procédé selon la revendication 11, dans lequel on répète successivement les étapes (a) à (d) en émettant des ondes ultrasonores focalisées différentes au cours des étapes d'excitation (a) successives, puis on combine les différentes cartographies obtenues au cours des étapes de cartographie (d) successives, pour calculer une cartographie synthétique du champ d'observation.

**13.** Dispositif d'imagerie utilisant des ondes de cisaillement pour observer un milieu viscoélastique diffusant (2) qui contient des particules (5) réfléchissant les ondes ultrasonores de compression, ce dispositif comprenant un réseau de transducteurs (6) commandés indépendamment les uns des autres par au moins une unité centrale électronique (4, CPU) adaptée pour la mise en oeuvre d'un procédé selon l'une quelconque des revendications précédentes.

**Claims**

**1.** An imaging method using shear waves for observing a diffusing viscoelastic medium (2) containing particles (5) that reflect ultrasound compression waves, said method comprising:

a) an excitation step during which an elastic shear wave is generated in the viscoelastic medium (2);
b) an observation step during which the propagation of the shear wave is observed simultaneously at a multitude of points in an observation field in the viscoelastic medium (2), this observation step comprising the following substeps:

b1) causing an array of transducers (6) that are controlled independently of one another to emit into the viscoelastic medium (2) a succession of unfocused ultrasound compression wave shots at a rate of at least 500 shots per second; and
b2) causing sound signals received from the viscoelastic medium (2) to be detected and recorded in real time, said signals comprising the echoes generated by the unfocused ultrasound compression wave interacting with the reflecting particles (5) in said viscoelastic medium; and

c) at least one processing step during which:

c1) the sound signals received successively from the viscoelastic medium (2) during substep b2) are processed in order to determine successive propagation images of the shear wave; and
c2) at least one movement parameter of the viscoelastic medium (2) is determined at different points of the observation field;

the method being **characterized in that** during excitation step a) the elastic shear wave is caused to be generated by causing at least one focused ultrasound wave to be emitted into the viscoelastic medium (2) by said array of transducers (6), the focusing and the timing of said focused ultrasound wave, and the timing of said unfocused ultrasound wave being adapted so that at least some of said unfocused ultrasound waves penetrate into the observation field while the shear wave is propagating in the observation field, for at least some of the unfocused ultrasound wave emissions.

2. A method according to claim 1, in which during substep b2), in order to determine said movement parameter, a plurality of successive propagation images are compared with a common reference image of the viscoelastic medium (2), the reference image being determined by firing at least one unfocused ultrasound compression wave into said viscoelastic medium and then detecting and recording echoes generated by said unfocused ultrasound compression wave on interacting with the reflecting particles (5) in the viscoelastic medium.

3. A method according to claim 2, in which step a) is preceded by an initial observation step a0) during which at least one unfocused ultrasound compression wave is fired and then echoes generated by said unfocused ultrasound compression wave interacting with the reflecting particles (5) in the viscoelastic medium are detected and recorded, said echoes corresponding to an initial image of the viscoelastic medium, and during substep b2), said initial image constitutes said reference image for processing at least some of the successive displacement images.

4. A method according to claim 3, in which, during initial observation step a0), a plurality of unfocused ultrasound compression waves are fired in succession and then echoes generated by each unfocused ultrasound compression wave interacting with the reflecting particles (5) of the viscoelastic medium are detected and recorded, said echoes corresponding to a plurality of successive images of the viscoelastic medium, and said initial image of the viscoelastic medium is determined by combining said successive images.

5. A method according to any preceding claim, in which said movement parameter is a displacement of the viscoelastic medium (2).

6. A method according to any preceding claim, in which the focused ultrasound wave emitted during excitation step a) presents a frequency $\underline{f}$ lying in the range 0.5 MHz to 15 MHz, and is emitted for a duration of k/f seconds, where $\underline{k}$ is an integer lying in the range 50 to 5000 and $\underline{f}$ is expressed in Hz.

7. A method according to any one of claims 1 to 5, in which the focused ultrasound wave emitted during excitation step a) presents a frequency lying in the range 0.5 MHz to 15 MHz and is emitted during a succession of emission periods separated by rest periods, the emission periods following one another at a rate lying in the range 10 to 1000 emissions per second.

8. A method according to any one of claims 1 to 5, in which the focused ultrasound wave emitted during excitation step a) is a linear combination (in particular a sum) of two monochromatic signals having respective frequencies f1 and f2 such that 20 Hz ≤ |f1 - f2| ≤ 1000 Hz.

9. A method according to any preceding claim, in which the focused ultrasound wave emitted during excitation step a) is focused simultaneously on a plurality of points.

10. A method according to any preceding claim, in which image processing step c) is followed by a mapping step d) during which, on the basis of variation in the movement parameter over time, at least one shear wave propagation parameter is calculated at at least some points of the observation field in order to determine a map of said propagation parameter in the observation field.

11. A method according to claim 10, in which the shear wave propagation parameter which is calculated during mapping step d) is selected from shear wave speed, shear modulus, Young's modulus, shear wave attenuation, shear elasticity, shear viscosity, and mechanical relaxation time.

12. A method according to claim 11, in which steps a) to d) are repeated successively while emitting different focused ultrasound waves during successive excitation step a), and then combining the maps obtained during the successive mapping step d) in order to calculate a combination map of the observation field.

13. Imaging apparatus using shear waves to observe a diffusing viscoelastic medium (2) containing particles (5) that reflect ultrasound compression waves, the apparatus comprising an array of transducers (6) that are controlled independently of one another by at least one electronic central unit (4, CPU) adapted for implementing a method according to any preceding claim.

**Patentansprüche**

1. Abbildungsverfahren mit Verwendung von Scherwellen, um ein viskoelastisches, streuendes Milieu (2) zu beobachten, das Partikel (5) enthält, welche die Ultraschalldruckwellen reflektieren, wobei das Verfahren umfasst:

(a) einen Anregungsschritt, in dessen Verlauf man eine elastische Scherwelle in dem viskoelastischen Milieu (2) erzeugt,
(b) einen Beobachtungsschritt, in dessen Verlauf man die Ausbreitung der Scherwelle gleichzeitig in einer Vielzahl von Punkten eines Beobachtungsfeldes in dem viskoelastischen Milieu (2) beobachtet, wobei dieser Beobachtungsschritt die folgenden Unterschritte umfasst:

(b1) Emittieren lassen einer nicht fokussierten Aufeinanderfolge von Schüssen von Ultraschalldruckwellen mit einem Takt von mindestens 500 Schüssen pro Sekunde in dem viskoelastischen Milieu (2) durch ein voneinander unabhängig gesteuertes Netz (6) an Wandlern,
(b2) Detektieren und Aufzeichnen lassen der vom viskoelastischen Milieu (2) empfangenen akustischen Signale in Echtzeit, umfassend die durch die nicht fokussierten Ultraschalldruckwellen während der Wechselwirkung mit den reflektierenden Partikeln (5) des besagten viskoelastischen Milieu erzeugten Echos,

(c) und mindestens einen Behandlungsschritt, in dessen Verlauf:

(c1) man die vom viskoelastischen Milieu (2) im Verlauf des Unterschritts (b2) erhaltenen aufeinander folgenden akustischen Signale verarbeitet, um aufeinander folgende Ausbreitungsbilder der Scherwelle zu bestimmen,
(c2) und man mindestens einen Bewegungsparameter des viskoelastischen Milieus (2) in verschiedenen Punkten des Beobachtungsfeldes bestimmt,

**dadurch gekennzeichnet, dass** man im Verlauf des Anregungsschrittes (a) die elastische Scherwelle erzeugen lässt, indem man mindestens eine fokussierte Ultraschallwelle in dem viskoelastischen Milieu (2) durch das besagte Wandlernetz (6) emittieren lässt, die Fokussierung und die zeitliche Aufeinanderfolge der besagten fokussierten Ultraschallwelle sowie die zeitliche Aufeinanderfolge der besagten nicht fokussierten Ultraschallwellen sind angepasst, damit mindestens einige der besagten nicht fokussierten Ultraschallwellen in dem Beobachtungsfeld bei der Ausbreitung der Scherwelle in diesem Beobachtungsfeld eintreffen, für mindestens einige Emissionen der nicht fokussierten Ultraschallwelle.

2. Verfahren gemäß Anspruch 1, in welchem man, um den besagten Bewegungsparameter zu bestimmen, im Verlauf des Unterschritts (b2) mehrere aufeinander folgende Ausbreitungsbilder mit einem selben Referenzbild des viskoelastischen Milieus (2) vergleicht, wobei dieses Referenzbild bestimmt wurde, indem mindestens eine nicht fokussierte Ultraschalldruckwelle in das besagte viskoelastische Milieu geschossen wurde, dann detektiert wird und die durch die besagte nicht fokussierte Ultraschalldruckwelle erzeugten Echos aufgezeichnet werden, wenn sie mit den reflektierten Partikeln (5) des viskoelastischen Milieus wechselwirkt.

3. Verfahren gemäß Anspruch 2, in welchem der Schritt (a) von einem initialen Beobachtungsschritt (a0) eingeleitet wird, in dessen Verlauf man mindestens eine nicht fokussierte Ultraschalldruckwelle abschießt, dann detektiert und die durch die besagte, nicht fokussierte Ultraschalldruckwelle erzeugten, während der Wechselwirkung mit den reflektierenden Partikeln (5) des viskoelastischen Milieus generierten Echos aufzeichnet, wobei diese Echos einem Initialbild des viskoelastischen Milieus entsprechen, und im Verlauf des Unterschritts (b2) bildet dieses besagte Initialbild das besagte Referenzbild, um mindestens einige der aufeinander folgenden Verschiebungsbilder zu verarbeiten.

4. Verfahren gemäß Anspruch 3, in welchem man im Verlauf des initialen Beobachtungsschritts (a0) mehrere nicht fokussierte Ultraschalldruckwellen aufeinander folgend abschießt, dann detektiert und die durch jede nicht fokussierte Ultraschalldruckwelle erzeugten, während der Wechselwirkung mit den reflektierenden Partikeln (5) des viskoelastischen Milieus generierten Echos aufzeichnet, wobei diese Echos in mehreren aufeinander folgenden Bildern dem viskoelastischen Milieu entsprechen, und man bestimmt das besagte Initialbild des viskoelastischen Milieus, indem man die besagten aufeinander folgenden Bilder kombiniert.

5. Verfahren gemäß einem der vorhergehenden Ansprüche, in welchem der besagte Bewegungsparameter eine Verschiebung des viskoelastischen Milieus (2) darstellt.

6. Verfahren gemäß einem der vorhergehenden Ansprüche, in welchem die im Verlauf des Anregungsschritts (a) emittierte fokussierte Ultraschallwelle eine zwischen 0,5 und 15 MHz gefasste Frequenz f darstellt und während einer Dauer k/f in Sekunden emittiert wird, wobei k eine ganze Zahl ist, gefasst zwischen 50 und 5000 und f in Hz ausgedrückt wird.

7. Verfahren gemäß einem der Ansprüche 1 bis 5, in welchem die im Verlauf des Anregungsschritts (a) emittierte fokussierte Ultraschallwelle eine zwischen 0,5 und 15 MHz gefasste Frequenz darstellt und während einer Aufeinanderfolge von durch Ruheperioden getrennten Emissionsperioden emittiert wird, wobei die Emissionsperioden aufeinander folgen mit einer Taktzeit zwischen 10 und 1000 Emissionen pro Sekunde.

8. Verfahren gemäß einem der Ansprüche 1 bis 5, in welchem die im Verlauf des Anregungsschritts (a) emittierte fokussierte Ultraschallwelle eine Linearkombination (insbesondere eine Summe) der beiden monochromatischen Signale der jeweiligen Frequenzen f1 und f2 ist, so dass 20 Hz ≤ |f1 - f2| ≤ 1000 Hz ist.

9. Verfahren gemäß einem der vorhergehenden Ansprüche, in welchem die im Verlauf des Anregungsschritts (a) emittierte fokussierte Ultraschallwelle gleichzeitig in mehreren Punkten fokussiert wird.

10. Verfahren gemäß einem der vorhergehenden Ansprüche, in welchem auf den Bildverarbeitungsschritt (c) ein Kartographieschritt (d) folgt, in dessen Verlauf man, beginnend mit einer Entwicklung des Bewegungsparameters im Verlauf der Zeit, mindestens einen Ausbreitungsparameter der Scherwelle in mindestens einigen Punkten des Beobachtungsfeldes berechnet, um so eine Kartographie des besagten Ausbreitungsparameters in dem Beobachtungsfeld zu bestimmen.

11. Verfahren gemäß Anspruch 10, in welchem der Ausbreitungsparameter der Scherwelle, der im Verlauf des Kartographieschritts (d) berechnet wird, unter der Geschwindigkeit der Scherwellen, dem Schermodul, dem Young'schen Modul, der Dämpfung der Scherwellen, der Scherelastizität, der Scherviskosität und der mechanischen Relaxationszeit gewählt wird.

12. Verfahren gemäß Anspruch 11, in welchem man die Schritte (a) bis (d) aufeinander folgend wiederholt, indem im Verlauf der aufeinander folgenden Anregungsschritte (a) verschiedene fokussierte Ultraschallwellen emittiert werden, dann die verschiedenen, im Verlauf der aufeinander folgenden Kartographieschritte (d) erhaltenen Kartographien kombiniert, um eine synthetische Kartographie des Beobachtungsfeldes zu berechnen.

13. Abbildungsvorrichtung mit Verwendung von Scherwellen, um ein viskoelastisches, streuendes Milieu (2) zu beobachten, das Partikel (5) enthält, die die Ultraschalldruckwellen reflektieren, wobei diese Vorrichtung ein Netz (6) von Wandlern umfasst, welche durch eine zentrale elektronische Verarbeitungseinheit (4, CPU) voneinander unabhängig gesteuert sind, angepasst für die Realisierung des Verfahrens gemäß einem der vorhergehenden Ansprüche.

# FIG.1.

**EP 1 546 757 B1**

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0055616 A
- WO 0232316 A
- FR 0210682

**Littérature non-brevet citée dans la description**

- **O'DONNELL et al.** Internal displacement and strain imaging using speckle tracking. *IEEE transactions on ultrasonic, ferroelectrics, and frequency control,* Mai 1994, vol. 41 (3), 314-325

- **OPHIR et al.** Elastography : a quantitative method for imaging the elasticity of biological tissues. *Ultrasonic imag.,* 1991, vol. 13, 111-134